(19) Europäisches Patentamt
European Patent Office
Office européen des brevets

(11) Veröffentlichungsnummer: **0 001 760**
**B1**

(12) **EUROPÄISCHE PATENTSCHRIFT**

(21) Anmeldenummer: **78101095.4**

(22) Anmeldetag: **07.10.78**

(51) Int. Cl.[3]: **C 07 C 121/45,**
C 07 D 239/42,
C 07 D 295/14

(54) **Alpha-aminomethylen-beta-formylaminopropionitril, Verfahren zu seiner Herstellung sowie Verwendung zur Herstellung von 2 - Methyl - 4 - amino - 5 - formylaminomethyl - pyrimidin**

(30) Priorität: **27.10.77 DE 2748153**
**26.04.78 DE 2818156**

(43) Veröffentlichungstag der Anmeldung:
**16.05.79 Patentblatt 79/10**

(45) Bekanntmachung des Hinweises auf die Patenterteilung:
**07.01.81 Patentblatt 81/01**

(84) Benannte Vertragsstaaten:
**BE CH DE FR GB NL**

(56) Entgegenhaltungen:
**DE - A - 2 323 845**

**TETRAHEDRON, vol. 27, 1971, Great Britain J.D. BONAFEDE et al "The bromination of enaminonitriles" Seite 3973 bis 3977**

**CHEMISCHE BERICHTE, Band 106, 1973 Weinheim, H. MORIMOTO et al. "Bildungsmechanismus von 4 - Amino - 5 - aminomethyl - 2 - methyl - pyrimidin aus 3 - Äthoxy - 2 - (diäthoxy - methyl) propionitril und Acetamidin" Seiten 893 bis 901**

(73) Patentinhaber: **BASF Aktiengesellschaft**
**Carl-Bosch-Strasse 38**
**D - 6700 Ludwigshafen (DE)**

(72) Erfinder: **Bewert, Wolfgang, Dr.**
**Lorscher Ring 8c**
**D - 6710 Frankenthal (DE)**
**Littmann, Wolfgang, Dr.**
**Neckarpromenade 36**
**D - 6800 Mannheim 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 001 760 B1

Alpha-aminomethylen-beta-formylaminopropionitril, Verfahren zu seiner Herstellung sowie Verwendung zur Herstellung von 2-Methyl-4-amino-5-formylaminomethyl-pyrimidin.

Die Erfindung betrifft N-substituiertes und unsubstituiertes $\alpha$ - Aminomethylen - $\beta$ - formylaminopropionitril, das zur Herstellung von 2 - Methyl - 4 - amino - 5 - formylaminomethyl - pyrimidin II dient, und seine Herstellung aus Metallsalzen des $\alpha$ - Formyl - $\beta$ - formylaminopropionitrils III. Die Verbindung II ist ein wichtiges Zwischenprodukt zur Herstellung von Vitamin $B_1$.

Aus der deutschen Offenlegungsschrift 23 23 845 ist bekannt, die Verbindung II durch Methylierung der Verbindung III mit Dimethylsulfat und Umsetzung des erhaltenen Enoläthers mit einem Amidin herzustellen.

Es wurde nun gefunden, daß man 2 - Methyl - 4 - amino - 5 - formylaminomethylpyrimidin der Formel II

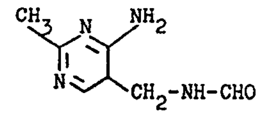

in einfacher Weise unter Vermeidung des giftigen Dimethylsulfats herstellen kann, wenn man Salze des $\alpha$ - Formyl - $\beta$ - formylaminopropionitrils III

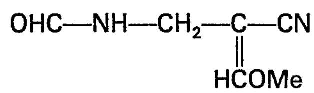

wobei Me ein Kation, vorzugsweise ein Alkali oder Erdalkalikation bedeutet, mit einem Salz des Ammoniaks oder eines Amins der Formel

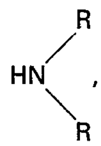 ,

in der die Reste R Wasserstoffatome oder gleiche oder verschiedene Alkyl, Aryl oder Aralkylreste bedeuten oder zusammen mit dem Stickstoffatom einen heterocyclischen Ring bilden können, zu den neuen Verbindungen $\alpha$ - Aminomethylen - $\beta$ - formylaminopropionitril der Formel I

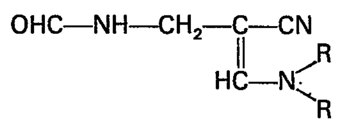

umsetzt, in der R die oben genannte Bedeutung hat und diese Verbindungen mit Acetamidin zum Pyrimidin II cyclisiert.

Zur Herstellung der Ausgangsverbindungen III wird auf die deutsche Offenlegungsschrift 23 23 845 verwiesen. Man erhält sie z.B. durch Umsetzung von $\beta$ - Aminopropionitril mit einem Ameisensäureester oder Kohlenmonoxid in Gegenwart von Metallalkoholat.

Die Metallsalze, insbesondere Alkalimetallsalze, des $\alpha$ - Formyl - $\beta$ - formylaminopropionitrils können dabei nach Isolierung oder auch in Form des Reaktionsgemisches, wie es aus der Herstellung ausgehend von $\beta$ - Aminopropionitril erhalten wird, zur Umsetzung mit den Salzen des Ammoniaks oder der Amine

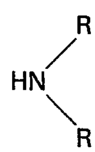

verwendet werden.

Als Verbindungen

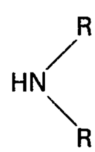

kommen dabei Ammoniak und primäre oder sekundäre, offenkettige oder cyclische Amine in Betracht. Im einzelnen seien Ammoniak, Monomethylamin, Monoäthylamin, Dimethylamin, Diäthylamin, n-Propylamin, iso - Propylamin, Dibutylamin, Morpholin, Piperidin, N - Methylanilin und insbesondere Anilin genannt. Es können beliebige weitere primäre oder sekundäre Amine verwendet werden, doch wird man sich aus Zweckmaßigkeitsgründen auf solche mit 1 bis 20, vorzugsweise 1 bis 10 Kohlenstoffatomen beschränken.

Man verwendet die Amine zweckmäßig in Form ihrer Salze, vorzugsweise der Salze von Halogenwasserstoffsäuren, insbesondere in Form der Hydrochloride. Man kann dabei auch zunächst das freie Amin in der Lösung der Verbindung III auflösen bzw. emulgieren und dann erst die Säure, insbesondere Salzsäure, zufügen.

In der Regel werden die Ammoniumsalze in stöchiometrischer oder nahezu stöchiometrischer Menge mit den Metallsalzen der Verbindungen II umgesetzt.

Man wendet zweckmäßig erhöhte Temperaturen bei der Umsetzung an, vorzugsweise erhitzt man auf die Siedetemperatur des verwendeten Lösungsmittels, bis ungefähr 1 Mol Wasser abgespalten ist.

Als Lösungsmittel kommen beispielsweise Alkohole wie Methanol, Kohlenwasserstoffe wie Benzol oder Chlorkohlenwasserstoffe wie Chloroform in Betracht. Demgemäß werden z.B. Temperaturen bis ungefähr 100°C angewandt. Die Umsetzung ist dann in einem kurzen Zeitraum, z.B. 15 bis 120 Minuten, beendet. Man erhält durch Filtration der heißen Lösung und Einengen des Filtrats das Enamin I.

Nach einer bevorzugten Ausführungsform der Erfindung wird das Aminhydrochlorid mit den Metallsalzen von $\alpha$ - Formyl - $\beta$ - formylaminopropionitril in wäßriger Lösung umgesetzt, wobei das entstandene Enamin mit einem mit Wasser nicht mischbaren, under den Reaktionsbedingungen inerten Lösungsmittel extrahiert wird.

Im einzelnen geht man dazu zweckmäßig so vor, daß man eine wäßrige Lösung des Aminohydrochlorids herstellt und in diese Lösung das Salz des $\alpha$ - Formyl - $\beta$ - formylaminopropionitrils einrührt. Es bildet sich eine klare Lösung, aus der das Produkt kontinuierlich extrahiert wird.

Die Reaktion verläuft so glatt, daß unmittelbar nach Zugabe des Propionitrils mit der Extraktion begonnen werden kann. Die Konzentration der wäßrigen Lösung ist nicht kritisch, doch empfiehlt es sich, nur mit solchen Mengen Wasser zu arbeiten, daß gerade eine vollständige Lösung der Salze gewährleistet ist.

Die Umsetzung geht bereits bei Raumtemperatur vonstatten. Allgemein ist ein Temperaturbereich von 20 bis 100°C anwendbar. Davon abweichende Temperaturen können gleichfalls angewendet werden, bringen jedoch keinen weiteren Vorteil.

Als zur Extraktion in Betracht kommende Lösungsmittel seien polare und unpolare Lösungsmittel wie Ester, aromatische Kohlenwasserstoffe oder Chlorkohlenwasserstoffe genannt. Im einzelnen sind Essigester, Methylenchlorid und Toluol zu nennen.

Durch Abdampfen des Extraktionsmittels erhält man das Enamin I bereits in guter Reinheit, das unmittelbar mit Acetamidin zum Pyrimidinderivat umgesetzt werden kann.

Nach einer weiteren Ausführungsform kann auf die Extraktion des Enamins verzichtet werden. In diesem Fall scheidet sich das Enamin als Öl in bereits weitgehend reiner Form ab und wird durch Kristallisation z.B. als Essigester oder Toluol, gereinigt.

Im einzelnen geht man zweckmäßig so vor, daß man eine wäßrige Lösung des Aminhydrochlorids herstellt und in diese Lösung des Salz des $\alpha$ - Formyl - $\beta$ - formylaminopropionitrils einrührt oder die wäßrige Aminhydrochloridlösung zu der wäßrigen Lösung des $\alpha$ - Formyl - $\beta$ - formylaminopropionitrils zutropft.

Man hält diese Mischung bei einer Temperatur von 40 bis 95°C, bevorzugt bei 80°C, und rührt 1 bis 20 Stunden, bevorzugt 6 Stunden. Nach Beendigung des Rührens findet in kürzester Zeit eine Phasentrennung statt, wobei die untere Phase aus Enamin besteht. Dieses kann abgelassen werden und aus den für die Extraktion genannten Lösungsmitteln umkristallisiert werden.

Die Umsetzung mit Acetamidin in Alkoholen wie Äthanol oder Methanol geschieht in der in der deutschen Offenlegungsschrift 23 23 845 für das Ausgangsmaterial $\alpha$ - Alkoxymethylen - $\beta$ - formylaminopropionitril beschriebenen Weise zu dem Pyrimidin II.

Das erhaltene Pyrimidin II entsteht in guter Ausbeute und ist unmittelbar zur Herstellung von Vitamin $B_1$ geeignet.

## Beispiel 1

82,2 Gewichtsteile Natriumsalze des $\alpha$ - Formyl - $\beta$ - formylaminopropionitrils (90 prozentig) werden in 550 Raumteilen Benzol suspendiert und mit 71,2 Teilen Anilinhydrochlorid 1 Stunde am Rückfluß gekocht, das Reaktionsgemisch heiß filtriert und auf die Hälfte eingeengt. Nach Isolieren und weiterem Einengen der Mutterlauge erhält man insgesamt 85,3 Teile $\alpha$ - Anilinnomethylen - $\beta$ - formylaminopropionitril vom Fp 119°C entsprechend 84,9% d. Th., bezogen auf das Na-Salz.

## Beispiel 2

74 Teile Natriumsalz des $\alpha$ - Formyl - $\beta$ - formylaminopropionitrils (75 prozentig) werden mit 58,3 Teilen Anilinhydrochlorid in 400 Raumteilen Methanol 1 Stunde am Rückfluß gekocht, das

Reaktionsgemisch filtriert und auf die Hälfte eingeengt. Man erhält 59,6 Teile, nach Einengen der Mutterlauge weitere 6,3 Teile entsprechend insgesamt 87,4% d.Th., bezogen auf das Na-Salz.

Beispiel 3

Man erhitzt 98,6 Teile Natriumsalze des $\alpha$ - Formyl - $\beta$ - formylaminopropionitrils (75 prozentig) mit 81,5 Teilen Dimethylammoniumchlorid in 700 Raumteilen Chloroform 1,5 Stunden am Rückfluß; es sind dann 8,5 Teile (theor. 9 Teile) Wasser abgeschieden. Man filtriert, engt das Filtrat zur Trockene ein und kristallisiert aus Methanol um. Es werden 43,0 Teile $\alpha$ - Dimethylaminomethylen - $\beta$ - formylaminopropionitril vom Fp 90°C erhalten.

In gleicher Weise werden erhalten

| Beispiel | Amin | gem. Beispiel | Fp | Ausbeute d. Th. |
|---|---|---|---|---|
| 4 | Morpholin-HCl | 3 | Öl | 56% |
| 5 | Diäthylamin-HCl | 1 | 56 - 58°C | 56,7% |
| 6 | n-Propylamin-HCl | 1 | Öl | 41,3% |
| 7 | Di-n-butylamin-HCl | 1 | Öl | 92% |
| 8 | N-Methylanilin-HCl | 1 | 80 - 82°C | 54,6% |

Beispiel 9

Man emulgiert 94 Teile Anilin in 500 Teilen Wasser und läßt zur Bildung des Hydrochlorids 230 Raumteile konzentrierte Salzsäure zulaufen. Dann rührt man 150 Teile Natriumsalz des $\alpha$ - Formyl - $\beta$ - formylaminopropionitrils (80 prozentig) bei 80°C ein und extrahiert die Lösung kontinuierlich mit Essigester. Nach dem Einengen der Essigesterphase fallen 139 Teile Enamin analysenrein an, d.s. 86% der Theorie.

Beispiel 10

Zu einer Lösung von 18 Teilen Acetamidin-hydrochlorid in 75 Raumteilen absolutem Äthanol gibt man 10,3 Gewichtsteile Natriummethylat, rührt die Mischung 30 Minuten bei Raumtemperatur, saugt von nicht gelösten Anteilen ab und versetzt mit 20,1 Teilen $\alpha$ - Anilinomethylen - $\beta$ - formylaminopropionitril gemäß Beispiel 2. Man rührt die Mischung 20 Minuten bei 25 bis 30°C und engt die entstandene Lösung zur Trockene ein. Das erhaltene Produkt wird mit 15 Raumteilen Methanol gewaschen und das Lösungsmittel abgesaugt.

Nach Wiederholung dieser Reinigungsoperation und Trocknen erhält man 11,1 Teile (ber. 100%) 2 - Methyl - 4 - amino - 5 - formylaminomethylpyrimidin.

Beispiel 11

Man emulgiert 94 Teile Anilin in 500 Teilen Wasser und läßt zur Bildung des Hydrochlorids 230 Raumteile konz. Salzsäure zulaufen.

Dann rührt man 150 Teile des Natriumsalzes des $\alpha$ - Formyl - $\beta$ - formylaminopropionitrils (80%ig) bei 80°C ein, rührt 4 Studen bei 80°C. Nach Eintritt der Phasentrennung läßt man die untere Phase ab und kristallisiert aus Essigester um. Man erhält 143 Teile Enamin, das sind 88,5% der Theorie.

Beispiel 12

Man löst 1 300 Teile des Natriumsalzes des $\alpha$ - Formyl - $\beta$ - formylaminopropionitrils (87%ig) in 3200 Teilen Wasser, emulgiert mit 745 Teilen Anilin und tropft bei 75°C 760 Raumteile konz. HCl zu. Man rührt 8 Stunden weiter bei 75°C, wartet dann die Phasentrennung ab und kristallisiert die untere Phase aus Toluol um. Man erhält 1245 g, das sind 81% der Theorie an Enamin.

**Patentansprüche**

1. $\alpha$ - Aminomethylen - $\beta$ - formylaminopropionitrile der Formel

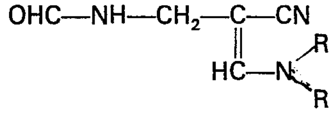

I,

in der die Rest R Wasserstoff oder gleiche oder verschiedene Alkyl-, Aryl- oder Aralkylreste bedeuten oder zusammen mit dem Stickstoffatom einen heterocyclischen Ring bilden.

2. Verfahren zur Herstellung von Verbindungen nach Anspruch 1, *dadurch gekennzeichnet*, daß man Metallsalze von $\alpha$ - Formyl - $\beta$ - formylaminopropionitril mit Salzen von Ammoniak oder Aminen der Formel

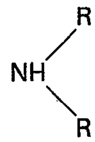

umsetzt, in der die Rest R die in Anspruch angegebenen Bedeutungen haben.

3. Verfahren gemäß Anspruch 2, *dadurch gekennzeichnet,* daß man $\alpha$ - Formyl - $\beta$ - formylamino-propionitril mit Salzen von Ammoniak oder Aminen in wäßriger Lösung umsetzt und das entstandene Enamin der Formel I isoliert.

4. Verfahren gemäß Anspruch 3, *dadurch gekennzeichnet,* daß man das entstandene Enamin mit einem mit Wasser nicht mischbaren, unter den Reaktionsbedingungen inerten Lösungsmittel extrahiert.

5. Verfahren gemaß Anspruch 3, *dadurch gekennzeichnet,* daß man das als Öl sich abscheidende Enamin abtrennt und durch Kristallisation reinigt.

6. Verwendung von Verbindungen der Formel

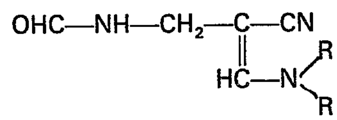

I,

in der die Reste R Wasserstoff oder gleiche oder verschiedene Alkyl-, Aryl- oder Aralkylreste bedeuten oder zusammen mit dem Stickstoffatom einen heterocyclischen Ring bilden können zur Herstellung der Verbindung der Formel

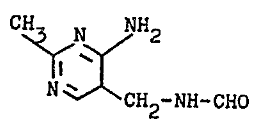

II,

durch Umsetzung mit Acetamiden.

**Revendications**

1. Alpha-aminométhylène-bêta-formylamino-propionitriles de la formule

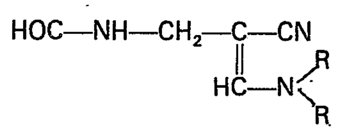

I,

dans laquelle les symboles R, qui peuvent être identiques ou différents, représentent chacun un atome d'hydrogène ou un radical alcoyle, aryle ou aralcoyle, ou forment avec l'atome d'azote adjacent un noyau hétérocyclique.

2. Procédé de préparation de composés suivant la revendication 1, caractérisé en ce que l'on fait réagir un sel de métal de l'alpha-formyl-bêta-formylamino-propionitrile avec un sel d'ammoniac ou d'amine de la formule

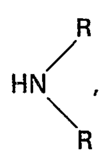

,

dans laquelle les symboles R possèdent la signification définie dans la revendication 1.

3. Procédé suivant la revendication 2, caractérisé en ce que l'on fait réagir l'alpha - formyl - bêta - formyl - amino - propionitrile avec un sel d'ammoniac ou d'amine en solution aqueuse, puis on isole l'énamine de la formule I formée.

4. Procédé suivant la revendication 3, caractérisé en ce que l'énamine formée est extraite par un solvant non miscible à l'eau et inerte dans les conditions réactionnelles.

5. Procédé suivant la revendication 3, caractérisé en ce que l'on isole l'énamine qui se sépare sous forme d'une huile et on la purifie par cristallisation.

6. Utilisation des composés de la formule

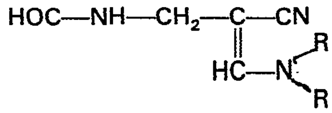

I,

dans laquelle les symboles R, qui peuvent être indentiques ou différents, représentent chacun un atome d'hydrogène ou un radical alcoyle, aryle ou aralcoyle, ou forment avec l'atome d'azote adjacent un noyau hétérocyclique, pour la préparation, par réaction avec l'acétamidine, du composé de la formule

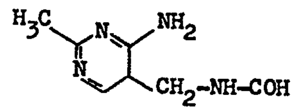

II,

## Claims

1. An $\alpha$-aminomethylene-$\beta$-formylaminopropionitrile of the formula

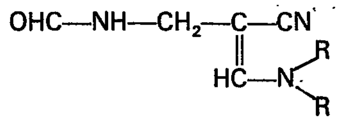

I,

where the radicals R are hydrogen or identical or different alkyl, aryl or aralkyl radicals or together with the nitrogen atom form a heterocyclic ring.

2. A process for the manufacture of a compound as claimed in claim 1, *characterized in that* a metal salt of $\alpha$-formyl-$\beta$-formylaminopropionitrile is reacted with a salt of ammonia or of an amine of the formula

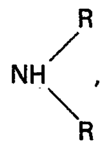

,

where the radicals R have the meanings given in claim 1.

3. A process as claimed in claim 2, *characterized in that* $\alpha$-formyl-$\beta$-formylaminopropionitrile is reacted with a salt of ammonia or of an amine in aqueous solution and the resulting enamine of the formula I is isolated.

4. A process as claimed in claim 3, *characterized in that* the resulting enamine is extracted with a water-immiscible solvent which is inert under the reaction conditions.

5. A process as claimed in claim 3, *characterized in that* the enamine which separates in the form of an oil is isolated and then purified by crystallization.

6. Use of a compound of the formula

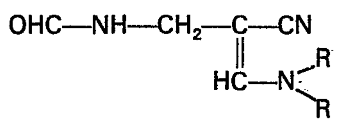

I,

where the radicals R are hydrogen or identical or different alkyl, aryl or aralkyl radicals or together with the nitrogen atom form a heterocyclic ring, for the manufacture of the compound of the formula

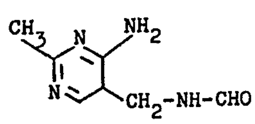

II,

by reaction with an acetamide.